(19) Europäisches Patentamt
European Patent Office
Office européen des brevets

(11) EP 2 056 099 B1

(12) **EUROPEAN PATENT SPECIFICATION**

(45) Date of publication and mention
of the grant of the patent:
**21.12.2016 Bulletin 2016/51**

(51) Int Cl.:
**G01N 27/406** (2006.01)   **G01K 1/20** (2006.01)
**G01N 33/00** (2006.01)

(21) Application number: **08018908.7**

(22) Date of filing: **29.10.2008**

(54) **Sensor control device**

Sensorsteuervorrichtung

Dispositif de contrôle du capteur

(84) Designated Contracting States:
**DE**

(30) Priority: **29.10.2007 JP 2007280993**

(43) Date of publication of application:
**06.05.2009 Bulletin 2009/19**

(73) Proprietor: **NGK Spark Plug Co., Ltd.**
**Nagoya-shi,**
**Aichi 467-8525 (JP)**

(72) Inventors:
• **Ishiguro, Yasuhiro**
**Nagoya-shi**
**Aichi 467-8525 (JP)**
• **Abe, Satoru**
**Nagoya-shi**
**Aichi 467-8525 (JP)**
• **Kobayashi, Akihiro**
**Nagoya-shi**
**Aichi 467-8525 (JP)**
• **Sumi, Takayuki**
**Nagoya-shi**
**Aichi 462-0809 (JP)**

(74) Representative: **Grünecker Patent- und**
**Rechtsanwälte**
**PartG mbB**
**Leopoldstraße 4**
**80802 München (DE)**

(56) References cited:
**EP-A- 0 859 232    EP-A- 0 937 979**
**US-A- 5 311 447**

**Description**

**BACKGROUND OF THE INVENTION**

1. Field of the Invention

The present invention relates to a sensor control device electrically connectable to a gas sensor and configured to drive-control the gas sensor, which gas sensor includes a detecting element for detecting specific gas components.

2. Description of the Related Art

As an example of a gas sensor for measuring specific gas components, an NOx sensor is known, configured to measure the concentration of NOx contained in exhaust gas. The NOx sensor includes a first oxygen pump cell and a second oxygen pump cell, each of which includes a solid electrolyte body. The first oxygen pump cell pumps oxygen into or out of exhaust gas introduced into a first chamber to adjust the concentration of oxygen contained in the first chamber. The first oxygen pump cell then transports oxygen originating from NOx contained in the exhaust gas into a second chamber. The concentration of NOx is detected based on current flowing through the second oxygen pump cell. The solid electrolyte body is non-conductive at room temperature, but is activated when the solid electrolyte body is heated so that the solid electrolyte body can transport oxygen. Therefore, the NOx sensor is provided with a heater element in order to heat the solid electrolyte bodies to an activation temperature.

The solid electrolyte body, which is activated by operation of the heater, has temperature dependent characteristics. Therefore, the solid electrolyte body may be thermally influenced by exposure to high-temperature exhaust gas in addition to the heating effect of an energized heater element. Since the current for detecting NOx concentration is very low, if the solid electrolyte body is thermally influenced by a periphery temperature which causes a variation in the resistance value thereof, the detection results may be inaccurate. JP-A-10-288595 describes a concentration detecting method for improving the concentration detecting precision. In the concentration detecting method, the temperature of a solid electrolyte body is detected, and a heating operation by a heater element is stabilized by executing feedback control. When there is a deviation between a target temperature and a detected temperature, the concentration detection results are corrected based on the temperature deviation so as to improve precision of the concentration detecting operation.

However, electronic components generally have temperature dependent characteristics at ambient temperatures. Therefore, in the case where electronic components mounted on the circuit board of the sensor control device for controlling sensors are thermally influenced at ambient temperatures, the detection results of a detection current in response to NOx concentration may deviate. More concretely, these electronic components are thermally influenced by heat transferred via circuit boards on which the electronic components are arranged. In other words, the electronic components may be thermally influenced by temperature variations occurring in the circuit boards. Circuits may be designed by using electronic components having less temperature dependent characteristics. However, since these electronic components are expensive, the cost of the device increases.

**SUMMARY OF THE INVENTION**

The present invention was made in consideration of the above circumstances, and an object thereof is to provide a sensor control device capable of detecting the concentration of a specific gas component with higher precision. The sensor control device corrects a detection result acquired by a gas sensor for the concentration of the specific gas component, in response to a detection result for a temperature of a circuit board acquired by a temperature sensing element mounted on the circuit board.

The present invention provides a sensor control device according to claim 1. Further aspects are covered in the dependent claims.

The concentration response signals outputted from the detecting element of the gas sensor are very low current signals on the order of milli-amperes (mA) or micro-amperes (pA), and the characteristics of the electronic components mounted on the circuit board can vary with a change in the temperature of the circuit board. When the electronic component characteristics fluctuate, accuracy in the detection result of the concentration of a specific gas component may be adversely affected. As a result, in the sensor control device of the present disclosure a temperature sensing element is provided on the circuit board, and a correcting process operation is carried out based on the detection result of the concentration of the specific gas in response to the detection result of the temperature (temperature information) of the circuit board. As a result, even when the characteristics of the electronic component provided on the circuit board vary with a change in the temperature of the circuit board so as to introduce an error in the detection result, the correct detection result of the concentration of the specific gas component can be obtained based on the correcting process operation. According to the present disclosure, even when electronic components having relatively large temperature dependent characteristics and relatively low cost are employed, the detection precision of the concentration of the specific gas component is not lowered, so that the manufacturing cost of the sensor control device can be reduced.

A relatively large current is supplied to the heater element which is provided in the gas sensor so as to heat the heater element. Accordingly, in the heater element driving unit, the electronic components provided on the circuit board may generate heat. Thus, the temperature sensing element is located closer to the detecting element driving unit than the heater element driving unit, or the temperature sensing element is arranged within the detecting element driving unit. Accordingly, the temperature of the circuit board can be correctly detected in order to compensate for the temperature dependency characteristics of the electronic components constituting the detecting element driving unit which handles very low current signals. Consequently, with respect to the gas concentration information (concentration response signal), errors which may arise in the detecting element driving unit due to the temperature of the circuit board can be accurately corrected, so that the precision of the detection result of the gas concentration can be improved.

As previously described, a sensor control device capable of increasing the detection precision of the specific gas component by the gas sensor may be employed in a gas sensor handling very small current signals. This sensor may be, for instance, an NOx sensor which generates a first concentration response signal as one of concentration response signals. The first concentration response signal reflects current flowing through the second oxygen pump cell in response to NOx concentration.

If the correcting process operation is carried out in consideration of the effect of the circuit board temperature on the electronic components, then the detection precision of the NOx concentration can be improved. In particular, in an NOx sensor containing a detecting element including a first oxygen pump cell and a second oxygen pump cell, the current flowing through the second oxygen pump cell has a magnitude on the order of $\mu$A. Consequently, if the correcting process operation is carried out in consideration of the effect of temperature, then very advantageous effects can be achieved.

In such an NOx sensor, a heater element for heating the detecting element may be provided. Thus, a heater element driving unit for energizing the heater element may be mounted on the circuit board on which the detecting element driving unit is mounted. However, since a relatively large current is supplied to heat the heating element, the electronic components provided on the circuit board in the heater element driving unit may generate a relatively large amount of heat. Also, in the NOx sensor including a first oxygen pump cell and a second oxygen pump cell, the current flowing through the second oxygen pump cell has a magnitude on the order of $\mu$A, which current is smaller than the current (normally, on the order of mA) flowing through the first oxygen pump cell. As a result, when the first cell control circuit is compared with the second cell control circuit, the current flowing through the energizing circuit of the second cell control circuit is easily influenced by a variation in the temperature of the circuit board.

As a consequence, in the case where the first and second cell control circuits constituting the detecting element driving unit are arranged on the circuit board, the second cell control circuit is located farther from the heater element driving unit than the first cell control circuit, and the temperature sensing element is located closer to the arranging position of the detecting element driving unit than the arranging position of the heater element driving unit, or the temperature sensing element is arranged within the detecting element driving unit. Since the second cell control circuit is located farther from the heater element driving unit than the first cell control circuit, the heat generated by the heater element driving unit hardly affects the second cell control circuit, so that temperature variations of the circuit board can be suppressed to a relatively small degree in the second cell control circuit. As a result, it is possible to reduce the adverse influence due to temperature variation of the circuit board, which is superimposed on the first concentration response signal. In addition, the temperature sensing element is located closer to the detecting element driving unit than the heater element driving unit, or the temperature sensing element is arranged within the detecting element driving unit. Accordingly, the temperature of the circuit board, to which the electronic components constituting the detecting element driving unit is subjected, is correctly detected. Consequently, it is possible to further improve precision in correcting errors due to temperature variation of the circuit board with respect to the first concentration response signal.

The NOx sensor comprising a detecting element including the first oxygen pump cell and the second oxygen pump cell may also include a second concentration response signal subject to temperature-correction, where the second concentration response signal represents a very small current which flows through the first oxygen pump in response to the oxygen concentration. Therefore, the detection precision of the oxygen concentration can be improved by performing the correcting process operation (in consideration of the adverse influence due to the temperature of the circuit board affecting the electronic components) with respect to the second concentration response signal.

The adverse influence of heat, to which the electronic components mounted on the circuit board are subjected, is greatly affected by the heat transfer (conduction) in the circuit board relative to the radiation heat radiated from other electronic components through the air. The temperature sensing element contacts the circuit board so as to detect the temperature of the circuit board. Accordingly, an error in the gas concentration information, due to the adverse influence imparted by the temperature of the circuit board to which the electronic components are subjected, can be precisely corrected. As a result, the concentration of a specific gas component can be detected with higher precision.

## BRIEF DESCRIPTION OF THE DRAWINGS

Fig. 1 is a schematic diagram showing an exhaust system of an internal combustion engine and peripheral elements thereof;

Fig. 2 is a schematic diagram showing a sensor control device according to an embodiment of the present invention and an NOx sensor connected to the sensor control device;

Figs. 3A and 3B are schematic diagrams showing a layout of electronic components mounted on a circuit board of the sensor control device according to the embodiment and another embodiment, respectively;

Fig. 4 is a flow chart describing a process operation for correcting NOx concentration of the present embodiment;

Fig. 5 is a graph illustrating a relationship between the temperature of the circuit board and detected NOx concentration values; and

Fig, 6 is a flow chart describing an NOx concentration correcting process operation according to another embodiment of the present invention.

## DETAILED DESCRIPTION OF THE PREFERRED EMBODIMENT

Embodiments of the present invention are next described with reference to the drawings. However, the present invention should not be construed as being limited thereto.

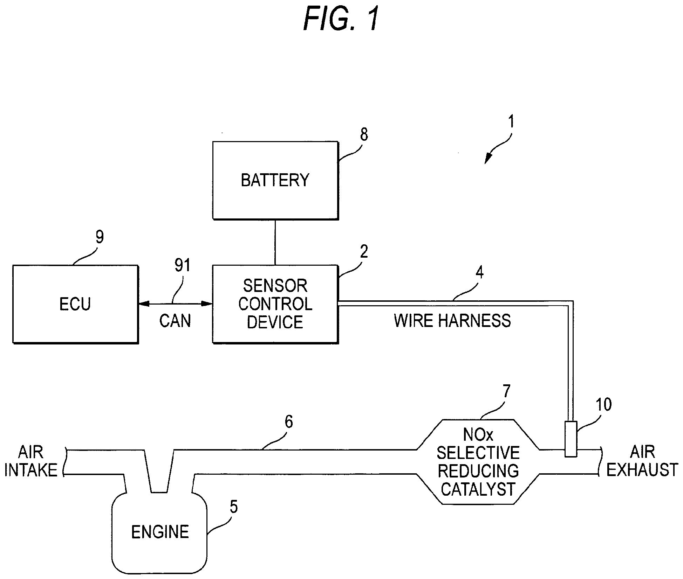

Firstly, a schematic structure of an internal combustion engine 1 including sensor control device 2 is described with reference to Fig. 1. The sensor control device 2 is an example of the sensor control device of the above aspects of the invention, and controls an NOx sensor 10 capable of sensing NOx concentration as a first specific gas component and oxygen concentration as a second specific gas component. In the following description, the "specific gas component" is also referred to as "specific gas." The first specific gas and the second specific gas correspond to the gas to be measured and are present in exhaust gas. Fig. 1 is a schematic diagram showing peripheral elements of the exhaust system of the internal combustion engine 1.

The internal combustion engine 1 of Fig. 1 includes an engine 5 used to drive an automobile. An exhaust pipe 6 is connected to the engine 5 in order to exhaust gas discharged from the engine 5 outside the automobile. An NOx selective reducing catalyst 7 for cleaning exhaust gas is provided in a flow path of the exhaust pipe 6. The NOx selective reducing catalyst 7 includes a catalyst in which exhaust gas reacts with an NOx reducing agent based upon a known chemical reaction so as to reduce NOx contained in the exhaust gas to harmless $H_2O$ and $N_2$. Although not shown in the drawing, an injector is provided on an upper stream side of the NOx selective reducing catalyst 7 (namely, the upper stream side of the flow path of the exhaust gas) which injects a urea solution into the exhaust gas flowing through the exhaust pipe 6.

The NOx sensor 10 is arranged on the down stream side of the NOx selective reducing catalyst 7 in the flow path of the exhaust pipe 6. The NOx sensor 10 is configured to detect the concentration of NOx contained in exhaust gas which passes through the NOx selective reducing catalyst 7. The NOx sensor 10 is electrically connected via a wire harness (signal line bundle) 4 to the sensor control device 2 which is remotely arranged at a position separated from the NOx sensor 10, and the NOx sensor 10 senses the concentration of NOx under control of the sensor control device 2. A battery 8 supplies electric power to the sensor control device 2 which is connected to an engine control unit (ECU) 9 via a controller area network (CAN, on-vehicle network) 91. The sensor control device 2 is configured to output a detection signal of the NOx concentration detected by the NOx sensor 10 to the ECU 9 via the CAN 91.

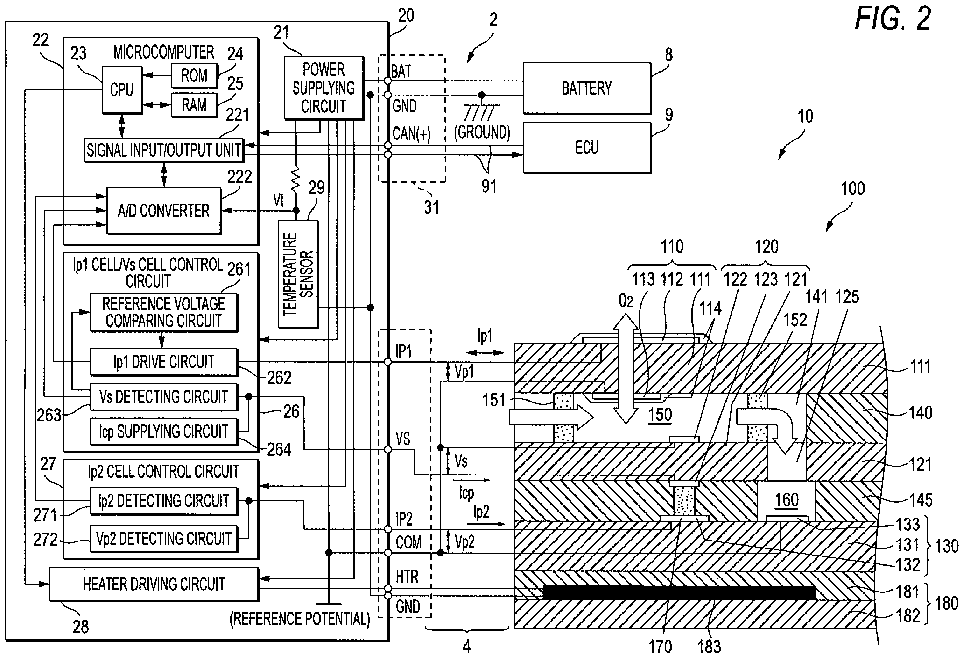

Next, the sensor control device 2 and the NOx sensor 10 are described in detail. Fig. 2 is a schematic diagram of the sensor control device 2 and the NOx sensor 10 connected to the sensor control device 2. In Fig. 2, a sensor element 100 of the NOx sensor 10 is illustrated by a cross-sectional view which shows its internal structure at a leading end portion. A right side of this cross-sectional view corresponds to leading end side of the sensor element 100.

In the NOx sensor 10 shown in Fig. 2, sensor element 100 having an elongated plate-shaped body is held inside a housing (not shown) which is provided for mounting the sensor element 100 to the exhaust pipe 6 (refer to Fig. 1). The wire harness 4 deriving a sensor signal from the sensor element 10 is drawn from the NOx sensor 10. The wire harness 4 is connected to a sensor terminal unit 30 of the sensor control device 2 located at a position separated from the NOx sensor 10, so that the NOx sensor 10 is electrically connected to the sensor control device 2.

Firstly, a description is made of the structure of the sensor element 100. The sensor element 100 has a layered structure in which insulating bodies 140 and 145 containing alumina are respectively sandwiched in spaces defined by two of the three sheets of plate-shaped solid electrolyte bodies 111, 121, 131. A heater element 180 is provided as an outer layer (namely, lower side as viewed in Fig. 1) on the solid electrolyte body 131 side of the sensor element. The heater element includes laminated insulating layers 181 and 182 and a heater pattern 183 mainly containing Pt (platinum) embedded in the laminated layers.

Each of the solid electrolyte bodies 111, 121, 131 contains zirconium (Zr) and has oxygen ion transferring characteristics. Electrodes 112 and 113 having a porous structure are respectively provided on opposing surfaces of the solid electrolyte body 111 along the laminating direction of the sensor element 100 so as to sandwich the solid electrolyte body 111. Each of the electrodes 112 and 113 contains Pt, a Pt alloy, or a cermet containing Pt and ceramic. Protection layers 114 having a porous structure and containing ceramic are provided on surfaces of the electrodes 112 and 113. Therefore, even if electrodes 112 and 113 are exposed to exhaust gas containing poison components, the protection layer 114 can prevent deterioration of the electrodes 112 and 113. The solid electrolyte body 111 and the solid electrolyte body 131 correspond to the "first solid electrolyte body" and "second solid electrolyte body," respectively, in the above aspects of the invention.

The solid electrolyte body 111 can pump oxygen into and out of (so-called "oxygen pumping") an atmosphere in contact with the electrode 112 (an atmosphere outside the sensor element 100), and another atmosphere in contact with the electrode 113 (namely, an atmosphere inside the first measuring chamber 150, described below), by supplying current across the electrodes 112 and 113. In the present embodiment, the solid electrolyte body 111 and the electrodes 112 and 113 are referred to as an "Ip1 cell 110." The Ip1 cell 110 corresponds to the "first oxygen pump cell", and the electrodes 112 and 113 correspond to "a pair of first electrodes" in the above aspects of the invention.

Next, the solid electrolyte body 121 is located opposite the solid electrolyte body 111 by sandwiching an insulating body 140 between the solid electrolyte bodies 121 and 111. Similarly, electrodes 122 and 123 having a porous structure are respectively provided on opposing surfaces of the solid electrolyte body 121 along the laminating direction of the sensor element 100 such that the electrodes 122 and 123 sandwich the solid electrolyte body 121. Each of the electrodes 122 and 123 contains Pt, a Pt alloy, or a cermet containing Pt and ceramic. The electrode 122 is formed on a surface facing to the solid electrolyte body 111.

A first measuring chamber 150 configured as a small space is defined between the solid electrolyte body 111 and the solid electrolyte body 121, in which the electrode 113 and the electrode 122 are arranged on a side of the solid electrolyte body 111 and provided on a side of the solid electrolyte body 121, respectively. This first measuring chamber 150 is a small space into which exhaust gas flowing through the exhaust pipe 6 (refer to Fig. 1) is firstly introduced within the sensor element 100. A first diffusion resisting portion 151 having a porous structure is provided on a leading end side of the sensor element 100 in the first measuring chamber 150 as a partition between the inside and outside of the first measuring chamber 150, and the first diffusion resisting portion 151 restricts the flow rate of exhaust gas into the first measuring chamber 150. Similarly, a second diffusion resisting chamber 152 is provided on the rear end side of the sensor element in the first measuring chamber 150 as a partition between the first measuring chamber 150 and an opening portion 141 connected to a second measuring chamber 160. The first measuring chamber 150 corresponds to the "first detecting chamber" in the above aspects of the invention.

The solid electrolyte body 121 and the electrodes 122 and 123 generate an electromotive force (EMF) in response to an oxygen partial pressure difference between atmospheres (namely, one atmosphere within the first measuring chamber 150 in contact with electrode 122, and another atmosphere within a reference oxygen chamber 170 in contact with electrode 123) isolated by the solid electrolyte body 121. The reference oxygen chamber 170 will be described below. In the present embodiment, the solid electrolyte body 121 and the electrodes 122 and 123 will be referred to as a "Vs cell" 120.

Insulating body 145 is sandwiched between the solid electrolyte body 131 and the solid electrolyte body 121. Similarly, electrodes 132 and 133 having a porous structure are provided on a surface of the solid electrolyte body 131 facing of the solid electrolyte body 121. Each of the electrodes 132 and 133 contains Pt, a PT alloy, or a cermet containing Pt and ceramic. The electrodes 132 and 133 correspond to the "a pair of second electrodes" in the above aspects of the invention.

At the position corresponding to the electrode 132, the insulating body 145 is not present, which defines the reference oxygen chamber 170 as an independent small space. The electrode 123 of the Vs cell 120 is arranged at the reference oxygen chamber 170. A porous body containing ceramic is filled into the reference oxygen chamber 170. At the position corresponding to the electrode 133, the insulating body 145 is not present, which defines a second measuring chamber 160 as an independent small space by isolating the insulating body 145 between the reference oxygen chamber 170 and the second measuring chamber 160. Opening portions 125 and 141 are formed in the solid electrolyte body 121 and the insulating body 140, respectively, which allow fluid communication with the second measuring chamber 160. The first measuring chamber 150 is connected to the opening portion 141 by sandwiching therebetween the second diffusion registering portion 152.

The solid electrolyte body 131 and the electrodes 132 and 133 can pump oxygen into or out of atmospheres (one atmosphere to which electrode 132 is exposed, and another atmosphere within second measuring chamber 160 in contact with the electrode 133) isolated by the insulating body 145. In this present embodiment, the solid electrolyte body 131 and the electrodes 132 and 132 are referred to as an "Ip2 cell 130." The Ip2 cell 130 corresponds to the "second oxygen pump cell" in the above aspects of the invention:

Next, a layout of the sensor control device 2 electrically connected to the sensor element 100 of the NOx sensor 10 is described. The sensor control device 2 includes a circuit board 20 having mounted thereon a microcomputer 22, an Ip1 cell/Vs cell control circuit 26, an Ip2 cell control circuit 27, a heater driving circuit 28 and a temperature sensor 29. Electric power is supplied to the circuits and circuit elements from a power supply circuit 21 also mounted on the circuit board 20. Battery power is supplied from a battery 8 connected to the power supply circuit 21 via a BAT port and a GND port of an external circuit terminal unit 31, such that the power supply circuit 21 can stabilize current and voltage which are supplied to the respective circuits.

The microcomputer 22 includes a CPU 23, a ROM 24, and a RAM 25. The microcomputer 22 is configured to communicate with the ECU 9 via the CAN 91 connected through a CAN (+) port and another CAN (-) port of the external circuit terminal unit 31. The microcomputer 22 further includes a signal input/output unit 221 and an A/D converter 222. The signal input/output unit 221 is connected via an A/D converter 222 to the Ip1 cell/Vs cell control circuit 26, the Ip2 cell control circuit 27 and the temperature sensor 29.

Under control of the microcomputer 22, the Ip1 cell/Vs cell control circuit 26, the Ip2 cell control circuit 27 and the heater driving circuit 28 perform a detecting operation of detecting NOx concentration contained in exhaust gas using the sensor element 100 of the NOx sensor 10. The Ip1 cell/Vs cell control circuit 26 includes a reference voltage comparing circuit 261, an Ip1 driving circuit 262, a Vs detecting circuit 263 and an Icp supplying circuit 264. The reference voltage comparing circuit 261 is configured to compare a voltage "Vs" between the electrodes 122 and 123 of the Vs cell 120 with a reference voltage (for instance, 425 mV) serving as a reference. The voltage "Vs" is detected by the Vs detecting circuit 263. The reference voltage comparing circuit 261 outputs a comparison result to the Ip1 driving circuit 262. The Ip1 driving circuit 262 is configured to supply current "Ip1" between the electrodes 112 and 113 of the Ip1 cell 110 connected via an IP1 port and a COM port of a sensor terminal unit 30. The IP1 driving circuit 262 adjusts the magnitude and flowing direction of the current "Ip1" based upon the output of the reference voltage comparing circuit 261. The Vs detecting circuit 263 is configured to detect the voltage "Vs" between the electrodes 122 and 123 connected via a VS port and a COM port of the sensor terminal unit 30. The Vs detecting circuit 263 outputs a detected value "Vs" to the reference voltage comparing circuit 261. The Icp supplying circuit 264 supplies current "Icp" between the electrodes 122 and 123 of the Vs cell 120, and pumps oxygen from the first measuring chamber 150 into the reference oxygen chamber 170. The electrode 113 of the Ip1 cell 110 disposed on the side of the first measuring chamber 150, the electrode 122 of the Vs cell 120 disposed on the side of the first measuring chamber 150, and the electrode 133 of an Ip2 cell 130 (described below) disposed on the side of the second measuring chamber 160 are connected to the reference potential via the COM port of the sensor terminal unit 30. The Ip1 cell/Vs cell control circuit 26 containing the Ip1 driving circuit 262 serves as the "first cell control circuit" in the above aspects of the invention.

The magnitude and the flow direction of the current "Ip1" are adjusted such that a voltage "Vs" between the electrodes 122 and 123 of the Vs cell 120 becomes substantially equal to a preset reference voltage based upon the comparison result of the voltage between the electrodes 122 and 123 of the Vs cell 120 obtained by the reference voltage comparing circuit 261. As a result, oxygen is pumped out of the first measuring chamber 150 to the outside of the sensor element 100 by the Ip1 cell 110, or oxygen is pumped from outside the sensor element 100 into the first measuring chamber 150 by the Ip1 cell 110. In other words, in the Ip1 cell 110, the concentration of oxygen within the first measuring chamber 150 is adjusted such that the voltage "Vs" between the electrodes 122 and 123 of the Vs cell 120 is maintained to a substantially constant value (namely, a reference voltage).

The Ip2 cell control circuit 27 includes an Ip2 detecting circuit 271 and a Vp2 applying circuit 272. The Ip2 detecting circuit 271 is configured to detect current "Ip2" which flows from the electrode 132 of the Ip2 cell 130 connected via the "IP2" port of the sensor terminal unit 30 to the electrode 133 connected via the COM port thereof. The Vp2 applying circuit 272 is configured to apply a voltage "Vp2" (for example, 450 mV) between the electrodes 132 and 133 of the Ip2 cell 130, such that oxygen is pumped into the reference oxygen chamber 170 from the second measuring chamber 160. The Ip2 cell control circuit 26 serves as the "second cell control circuit" in the above aspects of the invention.

The heater driving circuit 28 is controlled by the CPU 23 and configured to supply current to the heater pattern 183 of the heater element 180 so as to heat the solid electrolyte bodies 111, 121, and 131 (that is, the heater driving circuit 28 heats the Ip1 cell 110, the Vs cell 120 and the Ip2 cell 130). The heater driving circuit 28 performs a known PWM (pulse width modulation) control operation. That is, the heater driving circuit 28 supplies PWM-controlled current to the heater pattern 183 such that the temperatures of the solid electrolyte bodies 111, 121, 131 attain target temperatures. The heater pattern 183 is a single electrode pattern extending in the heater element 180, and one terminal portion thereof is grounded via a GND port of the sensor terminal unit 30, and the other terminal portion thereof is connected to the heater driving circuit 28 via an HTR port of the sensor terminal unit 30. The heater driving circuit 28 corresponds to the "heater element driving unit" in the above aspects of the invention.

The temperature sensor 29 configured to measure a temperature of the circuit board 20 is mounted on the circuit board 20 in the sensor control device 2 of the present embodiment. As the temperature sensor 29, for example, a chip resistor type thermistor is employed. One end of the temperature sensor 29 is grounded, and the other end thereof is connected to the power supply circuit 21 via a voltage dividing resistor 291, such that electric power is supplied to the temperature sensor 29. The resistance value of the temperature sensor 29 changes in response to the temperature of the circuit board 20, and a potential "Vt" at a voltage dividing point between the temperature sensor 29 and the voltage dividing resistor 291 changes in response to the temperature of the temperature sensor 29. The potential "Vt" of the voltage dividing point is inputted as a detection value of the temperature sensor 29 to the A/D converter 222 of the microcomputer 22 so as to be A/D-converted into the digital potential data which is entered to the CPU 23 via the signal input/output unit 221. The temperature sensor 29 corresponds to the "temperature sensing element", and the potential "Vt" corresponds to the "temperature response signal" in the above aspects of the invention.

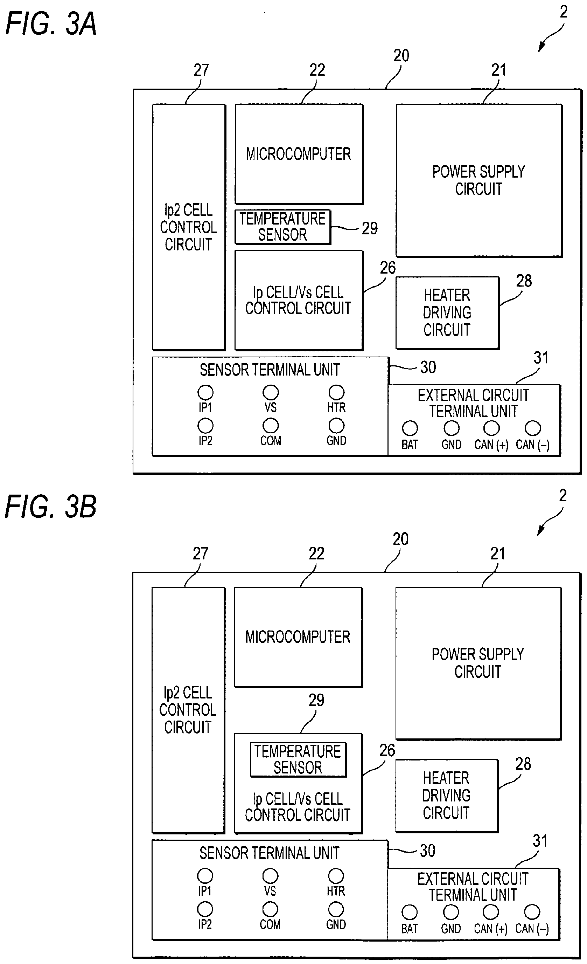

Fig. 3A is a schematic layout of the respective circuits of the sensor control device 2 and electronic components mounted on the circuit board 20.

As shown in Fig. 3A, the power supply circuit 21, the microcomputer 22, the Ip1 cell/Vs cell control circuit 26, the Ip2 cell control circuit 27, the heater driving circuit 28, the temperature sensor 29, the sensor terminal unit 30 and an ECU terminal unit (external circuit terminal unit) 31 are mounted on the circuit board 20 of the sensor control device 2. The sensor terminal unit 30 includes terminals (IP1, IP2, VS, COM, HTR and GND ports) provided along columns to which the respective lines of the wire harness 4 are connected to allow connection with the NOx sensor 10 (refer to Fig. 1). The sensor terminal unit 30 is arranged at one end portion on the board surface of the circuit board 20. The external circuit terminal unit 31 is arranged along the end side as this sensor terminal unit 30. In the external circuit terminal unit 31, respective terminals (CAN(+), CAN(-), BAT and GND ports) are provided along one column and allow connection with the CAN 91 for communication with the ECU 9, the signal lines from the battery 8 and the signal line for ground. For convenience, in the following description, the circuit board 20 is shown as having a rectangular plate shape having four edges corresponding to four sides of the rectangle, that is, an upper edge, a lower edge, a left edge and a right edge. The lower edge is defined as an edge where the sensor terminal unit 30 and the external circuit terminal unit 31 are arranged, whereas the upper edge is defined as an edge opposite the lower edge. Of the remaining two edges, the left edge is defined as an edge located close to the sensor terminal unit 30, and the right edge is defined as an edge located close to the external circuit terminal unit 31.

The Ip2 cell control circuit 27 is arranged along the left edge closer to the upper edge relative to the sensor terminal unit 30. The power supply circuit 21 and the heater driving circuit 28 are arranged at a position located on the right side closer to the upper edge relative to the external circuit terminal unit 31. The power supply circuit 21 is arranged closer to the upper edge relative to the heater driving circuit 28. The microcomputer 22 is arranged between the Ip2 cell control circuit 27 and the power supply circuit 21 at a position located on a side of the upper edge, and the Ip1 cell/Vs cell control circuit 26 is arranged between the microcomputer 22 and the sensor terminal unit 30. The Ip1 cell/Vs cell control circuit 26 is located adjacent to the Ip2 cell control circuit 27, and the Ip1 cell/Vs cell control circuit 26 is separated apart from the heater driving circuit 28. More specifically, in the present embodiment, the Ip1 cell/Vs cell control circuit 26 and the Ip2 cell control circuit 27 are mounted on the circuit board 20 such that the Ip2 cell control circuit 27 is separated apart from the heater driving circuit 28 with respect to the Ip1 cell/Vs cell control circuit 26. The temperature sensor 29 is arranged between the microcomputer 22 and the Ip1 cell/Vs cell control circuit 26. The temperature sensor 29 is also located adjacent to the Ip2 cell control circuit 27. In this embodiment, the Ip1 cell/Vs cell control circuit 26, the Ip2 cell control circuit 27, the power supply circuit 21 and the heater driving circuit 28 are mounted on a single surface of the circuit board 20. Alternatively, the mounting regions (arranging regions) of the respective circuits may be located on opposing surfaces of the circuit board 20 such that these mounting regions overlap with one another when the front surface and the rear surface of the circuit board 20 are viewed (i.e., in perspective plan view).

The sensor control device 2 detects the concentration of NOx contained in exhaust gas using the sensor element of the NOx sensor 10. Now, the following is a description of operations when the NOx concentration is detected by the NOx sensor 10. The solid electrolyte bodies 111, 121, 131, which configure the sensor element 100 of the NOx sensor 10 shown in Fig. 2, are heated by heater pattern 183 to which drive current is supplied from the heater driving circuit 28, so as to activate the solid electrolyte bodies 111, 121; 131. As a result, the Ip1 cell 110, the Vs cell 120, and the Ip2 cell 130 become operable.

Exhaust gas is introduced into the first measuring chamber 150 through the exhaust pipe 6 (refer to Fig. 1), while the flow rate of the exhaust gas within the sensor element is restricted by the first diffusion resisting portion 151. In this case, current "Icp" is supplied from the electrode 123 to the electrode 122 within the Vs cell 120 by the Icp supplying circuit 264. As a result, oxygen contained in the exhaust gas becomes oxygen ions, and the oxygen ions flow through the solid electrolyte body 121, and then, are moved into the reference oxygen chamber 170. In other words, since the current "Icp" flows between the electrodes 122 and 123, oxygen within the first measuring chamber 150 is transported into the reference oxygen chamber 170.

The Vs detecting circuit 263 detects a voltage between the electrodes 122 and 123, and the reference voltage comparing circuit 261 compares the detected voltage with a reference voltage (425 mV) and outputs the comparison result to the Ip1 driving circuit 262. In this case, when the concentration of oxygen present in the first measuring chamber 150 is adjusted such that a potential difference between the electrodes 122 and 123 becomes constant at approximately 425 mV, the concentration of oxygen contained in the exhaust gas within the first measuring chamber 150 may be approximated to a predetermined value (e.g., $10^{-8}$ to $10^{-9}$ atm).

In the case where the oxygen concentration of the exhaust gas introduced into the first measuring chamber 150 is lower than the predetermined value, the Ip1 driving circuit 262 supplies the current "Ip1" to the Ip1 cell 110 such that the electrode 112 assumes a negative polarity to pump oxygen from the outside of the sensor element 100 into the first measuring chamber 150. On the other hand, in the case where the oxygen concentration of the exhaust gas introduced into the first measuring chamber 150 is higher than the predetermined value, the Ip1 driving circuit 262 supplies the current "Ip1" to the Ip1 cell 110 such that the electrode 113 assumes a negative polarity to pump oxygen out from the first measuring chamber 150 to the outside of the sensor element 100. At this time, the oxygen concentration in the exhaust gas can be detected based upon a magnitude and a flow direction of the current "Ip1."

The exhaust gas where the oxygen concentration has been adjusted in the first measuring chamber 150 is introduced into the second measuring chamber 160 via the second diffusion resisting portion 152. NOx contained in the exhaust gas which contacts the electrode 133 in the second measuring chamber 160 is decomposed (reduced) into $N_2$ and $O_2$ by the electrode 133 acting as a catalyst. Then, the decomposed oxygen accepts electrons from the electrode 133 to become oxygen ions, and the oxygen ions flow through the solid electrolyte body 131 and thereafter are moved to the reference oxygen chamber 170. At this time, residual oxygen remaining in the first measuring chamber 150 is also moved to the reference oxygen chamber 170 by the Ip2 cell 130. As a result, current which flows through the Ip2 cell 130 becomes equal to a sum of the current originating from NOx and current originating from the residual oxygen. Since the concentration of the residual oxygen left in the first measuring chamber 150 during the pump-up operation has been adjusted to a predetermined concentration value the current originating from this residual oxygen is substantially constant. Therefore an adverse variation in the current originating from NOx is small. As a consequence, the variation of current flowing through the Ip2 cell 130 is substantially directly proportional to the NOx concentration. In the sensor control device 2, the current "Ip2" flowing through the Ip2 cell 130 is detected by the Ip2 detecting circuit 271, and a correction calculating operation for offset current originating from the residual oxygen, known by those of ordinary skill in this field of art, is performed based on the detected current value in order to detect the concentration of NOx contained in the exhaust gas. The current "Ip1" and "Ip2" (more precisely speaking, voltage signals converted from the current "Ip1" and "Ip2") correspond to the "first concentration response signal" and the "second concentration response signal," respectively, in the above aspects of the invention. Also, the current "Ip1" and "Ip2" correspond to the "concentration response signals" in the above aspects of the invention.

In the sensor control device 2 of the present embodiment, the temperature sensor 29 is provided on the circuit board 20 in order to detect the temperature of the circuit board 20, and the temperature sensor 29 is arranged at a position located close to the positions of the Ip1 cell/Vs cell control circuit 26 and the Ip2 cell control circuit 27. The current "Ip1" and "Ip2" handled by the Ip1 cell/Vs cell control circuit 26 and the Ip2 cell control circuit 27 are on order of mA and μA, respectively, i.e., very low currents. In the case where electronic components provided in these cell control circuits 26 and 27 are adversely influenced by a change in the temperature of the circuit board 20, an error may be introduced in detection values of the NOx concentration. For instance, in the case where a resistor is provided in a differential amplifying circuit, if a resistance value of the resistor changes depending on the temperature of the circuit board 20, the amplification factor of the differential amplifying circuit is changes. As a result, an error may be introduced in a detection result of the NOx concentration. On the other hand, in the heater driving circuit 28 and the power supply circuit 21, the currents that are handled are larger than the currents "Ip1" and "Ip2." Accordingly, even when the larger current changes with a change in the temperature of the circuit board 20, only a small error is introduced, which is hardly reflected onto the operating conditions and the operation results of the heater driving circuit 28 and the power supply circuit 21. Also, since these circuits 28 and 21 handle such large currents, electronic components employed in circuits 28 and 21 may generate heat. Consequently, in the present embodiment, the temperature sensor 29 is arranged at a position in the vicinity of those circuits readily influenced by the temperature of the circuit board 20 (for example, Ip1 cell/Vs cell control circuit 26, Ip2 cell control circuit 27, etc.), rather than other circuits which have a low temperature dependency (for instance, heater driving circuit 28, power supply circuit 21, etc.). Then, the temperature of the circuit board 20, which may adversely influence the electronic components constituting the Ip1 cell/Vs cell control circuit 26 and the Ip2 cell control circuit 27, is correctly detected. A correcting operation is carried out with respect to the detection value of the NOx concentration based upon a previously acquired relationship between the temperature of the circuit board 20 and the errors occurring in the detection value of the NOx concentration.

The current flowing through the first oxygen pump cell, i.e., the current "Ip1" handled by the Ip1 cell/Vs cell control circuit 26 is on the order of mA, whereas the current flowing through the second oxygen pump cell, namely the current "Ip2" handled by the Ip2 cell control circuit 27 is on the order of μA. That is, the current "Ip2" is smaller than the current "Ip1." Therefore, the smaller current "Ip2" rather than the current "Ip1" is more susceptible to a variation in temperature of the circuit board 20. In the embodiment, the Ip2 cell control circuit 27 is arranged on the circuit board 20. When viewed along the front surface of the circuit board 20, the Ip2 cell control circuit 27 is arranged at a position farther from the heater element driving circuit 28 than the arranging position of the Ip1 cell/Vs cell control circuit 26 (refer to Fig. 3A). As a result, heat generated by the heater driving circuit 28 hardly affects the Ip2 cell control circuit 27, and the adverse influence caused by temperature variation of the circuit board 20 can be suppressed in the Ip2 cell control circuit 27. In addition, the arranging position of the temperature sensor 29 is set to be close to the arranging positions of the Ip1 cell/Vs cell control circuit 26 and the Ip2 cell control circuit 27. Consequently, the precision of the correcting process operation with respect to the detection value of the NOx concentration can advantageously be maintained.

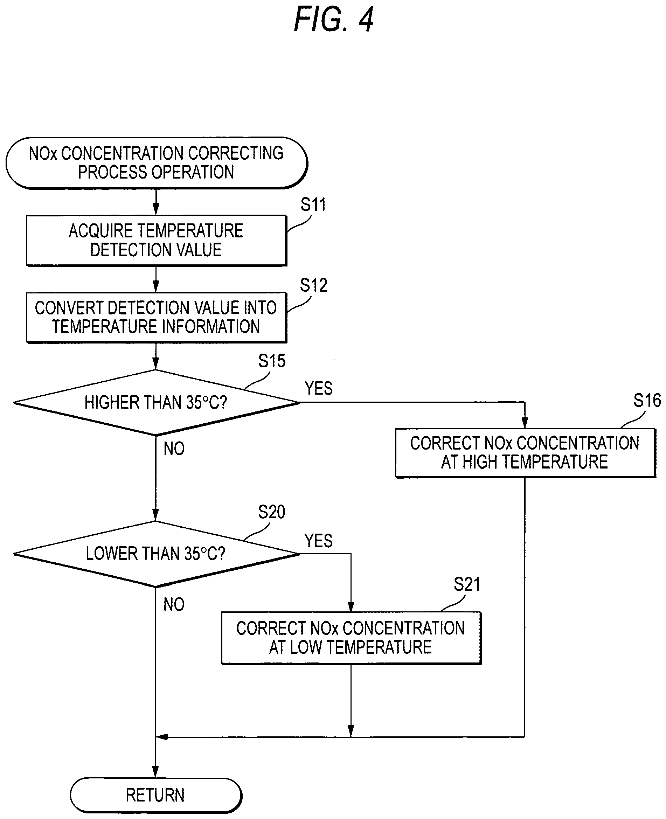

Referring now to Fig. 2 and Fig. 4, the following is a description of a correcting operation which is performed in response to a detection value of the temperature of the circuit board 20 with respect to the detection value of NOx concentration. Fig. 4 is a flow chart describing an NOx concentration correcting process operation. The NOx concentration correcting process operation is performed as one of subroutines called from a main program (not shown) which allows the sensor control device 2 to control the NOx sensor 10. The main program is previously stored on the ROM 24 of the microcomputer 22 and is executed by the CPU 23. The steps of the flow chart shown in Fig. 4 are described with abbreviated symbols "S."

The main program (not shown) for the sensor control device 2 is configured to manage timing and conditions at which the respective subroutines including the NOx concentration correcting process operation should be executed, and the CPU 23 executes the NOx concentration correcting processing operation indicated in Fig. 4 when the operation is called from the main progam. Before the NOx concentration correcting process operation is called, the process operation (not shown) for detecting the NOx concentration is called, and the detection value of the NOx concentration acquired by controlling the NOx sensor 10 in accordance with the detection operation is stored in the RAM 25.

As shown in Fig. 4, when the NOx concentration correcting process subroutine is called, the detection value of the temperature sensor 29 is firstly acquired (S11). Specifically, as shown in Fig. 2, the potential "Vt" produced at the junction of the temperature sensor 29 and the partial pressure resistor 291 changes, since the resistance value of the temperature sensor 29 changes in response to the temperature of the circuit board 20. This potential "Vt" (temperature response signal) is input as the detection value to the CPU 23 via the A/D converter 222.

The relationship between the detection value of the temperature sensor 29 and the temperature of the circuit board 20 is predetermined as a relation formula or a table by examination and the like, and is previously stored in the ROM 24. A detection value of the temperature sensor 29 is substituted for the formula or is converted by referring to the table, so that a temperature (temperature information) of the circuit board 20 detected by the temperature sensor 29 is acquired (S12).

Then, when the acquired temperature (temperature information) of the circuit board 20 is higher than 35°C ("YES" at S15), an NOx concentration correcting operation at a higher temperature is performed for the detection value of the NOx concentration stored on the RAM 25 (S16). This correcting operation is performed such that the temperature of the circuit board 20 and the detection value of the NOx concentration are substituted for the correction formula previously acquired and stored in the ROM 24 so as to calculate the correction value of the NOx concentration.

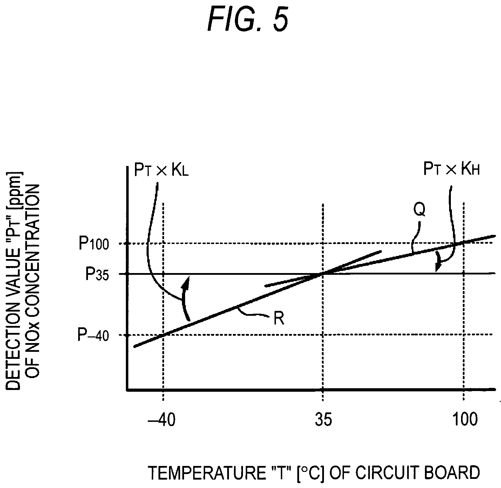

The correction formula is determined, for example, when a product checking operation is performed in accordance with the below-mentioned manner, and then, the correction formula thus obtained is stored in the ROM 24. That is, firstly, the circuit board 20 is heated (otherwise, cooled) such that the temperature of the circuit board 20 becomes 35°C, and a current of 0 $\mu$A is supplied to the IP2 port of the sensor terminal unit 30. In other words, the NOx concentration is brought into a pseudo-condition such that the concentration value thereof is 0 ppm. Under this pseudo-condition, the normal detecting process operation of the NOx concentration is performed to acquire a detection value "$P_{35}$" indicated in Fig. 5. Next, the circuit board 20 is heated to a temperature of 100°C. Similarly, under a condition in which a current of 0 $\mu$A is supplied to the IP2 port of the sensor terminal unit 30, a detecting process operation of NOx concentration is performed to acquire a detection value "$P_{100}$." Then, a relation formula "Q" (linear formula) is obtained from the relationship between the temperature of the circuit board 20 and the detection value of the NOx concentration thus obtained. In the relation formula "Q," the smaller the temperature dependent characteristic of the electronic components, the smaller the slope of the relation formula "Q". Consequently, by using the relation formula "Q", a formula for acquiring a correction coefficient "$K_H$" is derived. The correction coefficient "$K_H$" is a coefficient for correcting a detection value "$P_T$" of NOx concentration at the temperature of the circuit board 20 higher than 35°C so as to be coincident with a detection value "$P_{35}$" of the NOx concentration when the temperature of the circuit board 20 is 35°C. Then, "$P_T \times K_H$" is stored as a correction formula in the ROM 24.

In the correcting process operation for the NOx concentration at the high temperature of the circuit board 20 defined at step S16, the original detection value of the NOx concentration stored in the RAM 25 is overwritten by a correction value of the NOx concentration calculated by utilizing the correction formula "$P_T \times K_H$", and then the correcting process operation is accomplished. Thereafter, the process operation by the CPU 23 is returned to the main program (not shown).

When the temperature (temperature information) of the circuit board 20 acquired at step S12 is lower than 35°C ("NO" at S15, "YES" at S20), an NOx concentration correcting process operation at a lower temperature is performed for the detection value of the NOx concentration stored in the RAM 25 (S21). In this case, a correction formula "$P_T \times K_L$" is similarly determined during product inspection, and the correction formula thus obtained is previously stored in the ROM 24. In other words, a relation formula "R" is calculated using the detection values "$P_{35}$" and "$P_{-40}$" (refer to Fig. 5). The detection value "$P_{35}$" is acquired at a circuit board 20 temperature set to 35°C, and a detecting process operation of NOx concentration is performed under a condition in which a current of 0 $\mu$A is supplied to the IP2 port. The detection value "$P_{35}$" is acquired in the case where the temperature of the circuit board 20 is set to -40°C. Thereafter, by using the relation formula "R," a formula for acquiring a correction coefficient "$K_L$" is derived. The above-described correction coefficient "$K_L$" is a coefficient for correcting a detection value "$P_T$" of NOx concentration at a circuit board 20 temperature lower than 35°C to be coincident with a detection value "$P_{35}$" of NOx concentration at a circuit board 20 temperature of 35°C. "$P_T \times K_L$" is stored as a correction formula in the ROM 24. In a correcting process operation of the NOx concentration at low temperature in step S21, the original detection value of the NOx concentration stored in the RAM 25 is overwritten by a correction value of the NOx concentration calculated by utilizing the correction formula "$P_T \times K_L$", and then the correcting process operation is accomplished. Thereafter, the process operation by the CPU 23 is returned to the main program (not shown).

When a temperature (temperature information) of the circuit board 20 acquired at the step S12 is equal to 35°C ("NO" at S15, "NO" at S20), the correcting subroutine is directly returned to the main program (not shown) without executing the correcting process operation. In the main program, after the NOx concentration correcting process operation is performed, the corrected value of the NOx concentration stored in the RAM 25 is output to the ECU 9.

Accordingly, in the sensor control device 2 of the present embodiment, the temperature of the circuit board 20 is detected by the temperature sensor 29 provided on the circuit board 20. The error with respect to the detection value of the NOx concentration, due to the temperature dependent characteristics of the electronic components mounted on the circuit board 20, is corrected based upon the detection result of the temperature of the circuit board 20. Therefore, the detection precision of the NOx concentration can be increased. Moreover, the temperature sensor 29 is arranged, on the circuit board 20, closer to a circuit which is largely influenced by the temperature of the circuit board 20 (i.e., larger temperature dependency) than a circuit which is hardly influenced by the temperature of the circuit board 20. As a result, the temperature of the circuit board 20 at a position where the target circuit has been arranged can be correctly detected.

It should be understood that the present invention is not limited to the above-described embodiment, but that various modifications may be made within the spirit and scope of the claims appended hereto.

For instance, in the above embodiment, as shown in Fig. 3A, the temperature sensor 29 on the circuit board 20 is located close to the Ip1 cell/Vs cell control circuit 26 and the Ip2 cell control circuit 27. Alternatively, as shown in Fig. 3B, the temperature sensor 29 may be set within the Ip1 cell/Vs cell control circuit 26 and/or the Ip2 cell control circuit 27. In the above embodiment, those circuits which are hardly influenced by the temperature of the circuit board 20 (for example, the heater driving circuit 28, power supply circuit 21, etc.) are separated from those circuits which are largely influenced by the temperature of the circuit board 20 (for instance, the Ip1 cell/Vs cell control circuit 26, Ip2 cell control circuit 27 etc.). This layout of the electronic components on the circuit board 20 is preferable in that the temperature sensor 29 senses the temperature of the circuit board 20 at a position where a target circuit is arranged without interference from other circuits. In this case, when an imaginary line is drawn between the location of circuits which are hardly influenced by the temperature of the circuit board 20 and the location of circuits largely influenced by the temperature of the circuit board 20, the temperature sensor 29 may be positioned within, of two regions divided by the above imaginary line, a region where the circuits largely influenced by the temperature of the circuit board 20 are located.

Alternatively, a center position of the respective circuits may be identified, and the temperature sensor 29 may be located closer to the center position of those circuits largely influenced by the temperature of the circuit board 20 (for instance, the Ip1 cell/Vs cell control circuit 26, Ip2 cell control circuit 27, etc.) than the center position of those circuits hardly influenced by the temperature of the circuit board 20 (for example, the heater driving circuit 28, power supply circuit 21, etc.). The center position of these circuits may be determined in accordance with, for instance, the following method. Firstly, an arbitrary position on the circuit board 20 is determined as an origin, and then the locations of individual electronic components are located based upon absolute coordinates with respect to the origin. Thereafter, an average coordinate of all of the electronic components constituting a single circuit is calculated, and then the calculated average coordinate value is defined as a center position of the subject circuit. If this calculation method is employed, even when a part of the electronic components constituting one circuit is arranged in another circuit, the temperature sensor 29 can be located on the side of a circuit which reflects an influence of environmental temperature so that the temperature of the circuit board 20 can be correctly detected.

In the NOx concentration correcting process operation described above, the temperature of 35°C is defined as a reference temperature, the environmental temperatures are subdivided into two cases, higher than 35°C and lower than 35°C, and then, the detection values of the NOx concentration are corrected by applying the corresponding correction formulae. This temperature condition is only one example. Accordingly, the reference temperature for subdividing the cases is not be limited to 35°C. Also, as to the temperatures at which the NOx concentration of the reference gas is detected when the correction formula at a high temperature and the correction formula at the low temperature are determined, the present invention is not limited to 100°C and -40°C, respectively. Alternatively, the relationship between the temperature of the circuit board 20 and corresponding correction coefficients may be stored in the form of a data table instead of correction formulae, and the correction value may be calculated by multiplying the detection value of the original NOx concentration by a coefficient acquired by referring to the data table in response to the temperature of the circuit board 20.

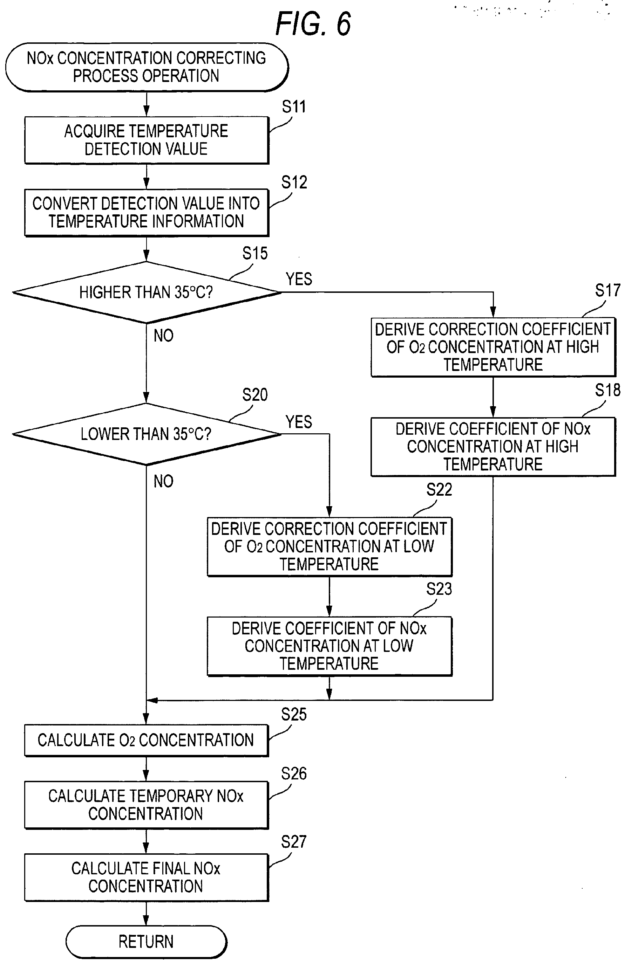

Also, in the above embodiment, as one example, the detection value of the NOx concentration acquired after executing the program is corrected in response to the temperature of the circuit board 20. Alternatively, the correcting process operation may be performed for a value obtained upon converting the current "Ip2" into digital data (hereinafter referred to as "Ip2 digital value"), and prior to outputting the detection value to the ECU 9 in response to the temperature information of the circuit board 20. Similarly, the correcting process operation may be performed in response to temperature information of the circuit board 20 not only for the NOx concentration, but also for a detection value of oxygen concentration acquired based on the current "Ip1" (second concentration response signal). In that case, data obtained before the concentration is converted as a detection value of the oxygen concentration, i.e., a value obtained by converting the current "Ip1" into digital data (hereinafter referred to as "Ip1" digital value") is subjected to the correcting process operation. Referring now to a flow chart of Fig. 6, the following is a description of one example of a correcting method.

In an NOx concentration correcting process operation which is represented as a modification in Fig. 6, the correcting process operations are performed for each of digital data, namely, an Ip1 digital value and an Ip2 digital value, which are obtained by A/D-converting the current "Ip1" and the current "Ip2" (precisely speaking, voltage signals produced by I/V-converting currents "Ip1" and "Ip2"), while the currents "Ip1" and "Ip2" are output from the Ip1 cell/Vs cell control circuit 26 and the Ip2 cell control circuit 27 to the microcomputer 22. The Ip1 digital value and the Ip2 digital value are acquired by executing on other program (not shown) called from the main routine and stored in the RAM 25. When the NOx concentration correcting process operation of the modification is called from the main program (not shown), a detection value of the temperature sensor 29 is firstly acquired similar to the above embodiment (S11), and the acquired detection value is converted into a temperature (temperature information) of the circuit board 20 (S12).

When the temperature (temperature information) of the circuit board 20 is higher than 35°C ("YES" at S15), first of all, a correction coefficient "$\alpha_H$" of oxygen concentration at a high temperature is derived (S17). The correction coefficient "$\alpha_H$" may be calculated as a relation formula with respect to the temperature of the circuit board 20 similar to the method for calculating the correction coefficient "$K_H$" during the production inspection in the above embodiment and may be previously stored in the ROM 24. Concretely, under the condition where the temperature of the circuit board 20 is set to 35°C and where a current of 0 $\mu$A is supplied from an external source to the IP1 port, a relation formula (not shown) is obtained by employing an Ip1 digital value "$U_{35}$" and another Ip1 digital value "$U_{100}$." The Ip1 digital value "$U_{35}$" is obtained by entering the current "Ip1" via the Ip1 cell/Vs cell control circuit 26 to the microcomputer 22 and by A/D-converting the entered current "Ip1." The Ip1 digital value "$U_{100}$" is obtained by processing the current "Ip1" when the temperature of the circuit board 20 is set to 100°C. Then, a formula is derived from the calculated relation formula and is stored in the ROM 24, while this formula is provided in order to calculate the correction coefficient "$\alpha_H$." This correction coefficient "$\alpha_H$" is utilized so that an Ip1 digital value "$U_T$" at an arbitrary temperature "T" at the temperature of the circuit board 20 higher than 35°C may be coincident with another Ip1 digital value "$U_{35}$" at the temperature of the circuit board 20 of 35°C. At step S17, the correction coefficient "$\alpha_H$" is derived by substituting the temperature of the circuit board 20 acquired at step S12 into the formula, and the "$\alpha_H$" is stored in the RAM 25.

Next, a correction coefficient "$\beta_H$" for the NOx concentration at the high temperature is derived (S18). The correction coefficient "$\beta_H$" may be calculated by executing a method similar to the above-described calculating method for obtaining the correction coefficient "$\alpha_H$" during the product inspection, and the calculated correction coefficient "$\beta_H$" may be previously stored in the ROM 24. That is, under the condition where a current of 0 $\mu$A is supplied to the IP2 port, an Ip2 digital value "$V_{35}$" at a temperature of the circuit board 20 of 35°C and another Ip2 digital value "$V_{100}$" at a temperature of the circuit board 20 of 100°C is determined; a relation formula of a Ip2 digital value "$V_T$" at an arbitrary temperature with respect to a temperature "T" of the circuit board 20 is calculated; and the formula for calculating the correction coefficient "$\beta_H$" is derived and is stored in the ROM 24. The correction coefficient "$\beta_H$" is utilized so that the Ip2 digital value "$V_T$" may be coincident with the Ip2 digital value "$V_{35}$" at the temperature of the circuit board 20 of 35°C. At step S18, the correction coefficient "$\beta_H$" is derived by substituting the temperature of the circuit board 20 acquired at step S12 into the formula, and the derived "$\beta_H$" is stored in the RAM 25. Thereafter, the correcting process operation proceeds to the step S25.

. When the temperature (temperature information) of the circuit board 20 is lower than 35°C ("NO" at S15, and "YES" at S20), a correction coefficient "$\alpha_L$" of oxygen concentration at a low temperature is derived (S22), and another correction coefficient "$\beta_L$" of NOx concentration at the low temperature is determined (S23). The correction coefficient "$\alpha_L$" may be calculated by executing a method similar to the above-described calculating method during the product inspection, and then, the calculated correction coefficient "$\alpha_L$" may be previously stored in the ROM 24. That is, under the condition where a current of 0 $\mu$A is supplied to the IP1 port, an Ip2 digital value "$U_{35}$" at a temperature of the circuit board 20 of 35°C and another Ip2 digital value "$U_{-40}$" at a temperature of the circuit board 20 of -40°C is determined; a relation formula of a Ip1 digital value "$U_T$" at an arbitrary temperature lower than 35°C with respect to a temperature "T" of the circuit board 20 is calculated; and the formula for calculating the correction coefficient "$\alpha_L$" is derived and is stored in the ROM 24. The correction coefficient "$\alpha_L$" is utilized so that the Ip1 digital value "$U_T$" may be coincident with the Ip1

digital value "$U_{35}$" at a temperature of the circuit board 20 of 35°C. Similarly, the correction coefficient "$\beta_L$'" may be calculated as follows: That is, under the condition where a current of 0 μA is supplied to the Ip2 port during the product inspection, an Ip2 digital value "$V_{35}$" at a temperature of the circuit board 20 of 35°C and another Ip2 digital value "$U_{-40}$" at a temperature of the circuit board 20 of -40°C are acquired; a relation formula of an Ip2 digital value "$V_T$" at an arbitrary temperature lower than 35°C with respect to the temperature "T" of the circuit board 20 is acquired; and the formula for calculating the correction coefficient "$\beta_L$" is derived and stored in the ROM 24. The correction coefficient "$\beta_L$" is utilized so that the Ip2 digital value "$V_T$" may be coincident with the Ip2 digital value "$V_{35}$" at a temperature of the circuit board 20 of 35°C. The correction coefficient "$\alpha_L$" derived at step S22 and the correction coefficient "$\beta_L$" derived at step S23 are stored in the RAM 25. Thereafter, the correcting process operation proceeds to step S25.

In the case where the temperature (temperature information) of the circuit board 20 acquired at step S12 is 35°C ("NO" at S15, "NO" at S20), the correcting process operation directly proceeds to step S25 without obtaining a correction coefficient.

Next, at step S25, the oxygen concentration is calculated based on the following formula (1) (step S25).

$$\text{(oxygen concentration)} = \text{(Ip1 digital value "}U_T\text{")} \times \text{(Ip1 gain value)} - \text{(Ip1 offset value)} \times \text{(correction coefficient "}\alpha_H\text{" (otherwise, correction coefficient "}\alpha_L\text{"))} \quad --- (1)$$

The Ip1 gain value and the Ip1 offset value correspond to previously set values obtained during product inspection in order to correct individual differences among plural sensor elements 100.

Subsequently, a temporary value of NOx concentration (namely, temporary NOx concentration) is calculated based on the following formula (2) (step S26).

$$\text{(temporary NOx concentration)} = \text{(Ip2 digital value "}V_T\text{")} \times \text{(Ip2 gain value)} - \text{(Ip2 offset value)} \times \text{(correction coefficient "}\beta_H\text{" (otherwise, correction coefficient "}\beta_L\text{"))} \quad --- (2)$$

The Ip2 gain value and the Ip2 offset value correspond to previously set values obtained during product inspection in order to correct individual differences among plural sensor elements 100.

Then, a final detection value of NOx concentration (final NOx concentration) to be outputted to the ECU 9 is calculated by employing the oxygen concentration calculated at step S25 and the temporary NOx concentration calculated at step S26 based upon the following formula (3) (step S27).

$$\text{(final NOx concentration)} = \text{(temporary NOx concentration)} / \text{((oxygen concentration)} \times X + Y) \quad --- (3)$$

Symbols "X" and "Y" are predetermined coefficients larger than zero. The final NOx concentration calculated above is stored in the RAM 25, and then the correcting process operation is once returned to the main program (not shown) Thereafter, this final NOx concentration is output to the ECU 9 as the detection value of the NOx concentration.

The above-described modification to the correcting process operation for the NOx concentration is directed to an example in which the correcting process operation is carried out for the Ip1 offset value and the Ip2 offset value. Alternatively, a correcting process operation may be performed by considering the effect of temperature of the circuit board 20 on the Ip1 and Ip2 gain values. In this alternative case, correction coefficients to be multiplied by the Ip1 and Ip2 gain values may be calculated by the following method. That is, the temperature of the circuit board 20 may be set under a condition where the current flowing through the IP1 port is different from the current flowing through the IP2 port as described above to obtain Ip1 and Ip2 digital values, and the correction coefficients may be calculated based on the Ip1 and Ip2 digital values thus obtained.

A chip resistor type thermistor is illustrated as one example of the temperature sensor 29. Alternatively, a thermocouple, a platinum temperature measuring resistance member, and the like may be employed as the temperature sensor 29. Furthermore, in detecting the temperature of the circuit board 20, the temperature sensor 29 preferably is in contact with the circuit board 20 so as to utilize a thermal transfer operation. This results in precise detection of the temperature of the circuit board 20. Although the sensor control device 2 of the present embodiment is exemplified as the control device for the NOx sensor 10, the sensor control device 2 may be alternatively applied to other gas sensors, for instance, oxygen sensors, HC sensors, and the like.

This application is based on Japanese patent Application No. 2007-280993 filed October 29, 2007.

**Claims**

1.  A sensor control device comprising:

    a circuit board (20) separated from and electrically connectable to a gas sensor (10), said gas sensor (10) comprising a detecting element (100) configured to output a concentration response signal in response to the concentration of a specific gas component, and a heater element (180) configured to activate the detecting element;
    a detecting element driving unit (26, 27, 22) mounted on the circuit board (20), the detecting element driving unit (26, 27, 22) being configured to:

        control the gas sensor (10);
        calculate gas concentration information based on the concentration response signal; and
        output the calculated gas concentration information to an external circuit; and

    a heater element driving unit (28) mounted on the circuit board (20) and configured to supply driving current to the heater element (180),

    **characterized by**

    a temperature sensing element (29) mounted on the circuit board (20) and configured to output a temperature response signal in response to a temperature of the circuit board (20);
    a temperature calculating unit (23) mounted on the circuit board (20) and configured to calculate temperature information of the circuit board (20) based on the temperature response signal; and
    a concentration information correcting unit (23) mounted on the circuit board (20) and configured to correct the gas concentration information calculated by the detecting element driving unit (26, 27, 22) based on the temperature information calculated by the temperature calculating unit (23),

    wherein the temperature sensing element (29) is located closer to the detecting element driving unit (26, 27, 22) than to the heater element driving unit (28), or the temperature sensing element (29) is arranged within the detecting element driving unit (26, 27, 22).

2.  The sensor control device according to claim 1,
    wherein the gas sensor (10) is an NOx sensor configured to sense NOx concentration as a first specific gas component contained in a gas to be measured, wherein the detecting element comprises:

    a first detecting chamber (150) in which the gas to be measured is introduced via a first diffusion resisting portion (151);
    a first oxygen pump cell (110) comprising a first solid electrolyte body (111) and a pair of first electrodes (112, 113) formed on the first solid electrolyte body, one of the pair of first electrodes being arranged inside the first detecting chamber, the first oxygen pump cell being configured to pump oxygen into or out of the gas to be measured introduced into the first detecting chamber; (150)
    a second detecting chamber (160) in which gas from the first chamber is introduced via a second diffusion resisting portion (152); and
    a second oxygen pump cell (130) comprising a second solid electrolyte body (131) and a pair of second electrodes (132) formed on the second solid electrolyte body, one of the pair of second electrodes being arranged inside the second detecting chamber (160),

    wherein the concentration response signal is a first concentration response signal obtained based on current flowing through the second oxygen pump cell (130) in response to the concentration of NOx in the second detecting chamber (160).

**3.** The sensor control device according to claim 2,
wherein the detecting element driving unit is located on the circuit board separate from the heater element driving unit;
wherein the detecting element driving unit comprises:

a first cell control circuit (26) configured to apply a voltage to the first oxygen pump cell so as to supply current to the first oxygen pump cell (110); and

a second cell control circuit (27) located farther from the heater element driving unit (28) than the first cell control circuit and configured to apply a voltage to the second oxygen pump cell so as to supply current to the second oxygen pump cell (130).

**4.** The sensor control device according to claim 2 or 3,
wherein the concentration response signal includes a second concentration response signal in addition to the first concentration response signal, and
wherein when oxygen is pumped into or out of the gas to be measured introduced into the first detecting chamber (150) by the first oxygen pump cell (110), the second concentration response signal is obtained based on the current flowing through the first pump cell in response to the concentration of oxygen as a second specific gas component contained in the gas to be measured.

**5.** The sensor control device according to any of claims 1 to 4,
wherein the temperature sensing element (29) contacts the circuit board (20) to detect the temperature of the circuit board (20).

**Patentansprüche**

**1.** Sensor-Steuerungsvorrichtung, die umfasst:

eine Leiterplatte (20), die von einem Gassensor (10) getrennt ist und elektrisch mit ihm verbunden werden kann, wobei der Gassensor (10) ein Erfassungselement (100), das so ausgeführt ist, dass es in Reaktion auf die Konzentration einer bestimmten Gaskomponente ein Konzentrations-Reaktionssignal ausgibt, sowie ein Heizelement (180) umfasst,
das so ausgeführt ist, dass es das Erfassungselement aktiviert;
eine Einheit (26, 27, 22), zum Ansteuern des Erfassungselementes, die an der Leiterplatte (20) montiert ist, wobei die Einheit (26, 27, 22) zum Ansteuern des Erfassungselementes so ausgeführt ist, dass sie:

den Gassensor (10) steuert;
Gaskonzentrations-Informationen auf Basis des Konzentrations-Reaktionssignals berechnet; und
die berechneten Gaskonzentrations-Informationen an eine externe Schaltung ausgibt; sowie

eine Einheit (28) zum Ansteuern des Heizelementes, die an der Leiterplatte (20) montiert ist und so ausgeführt ist, dass sie dem Heizelement (180) Ansteuerstrom zuführt,

**gekennzeichnet durch**

ein Temperatur-Messelement (29), das an der Leiterplatte (20) montiert und so ausgeführt ist, dass es in Reaktion auf eine Temperatur der Leiterplatte (20) ein Temperatur-Reaktionssignal ausgibt;
eine Temperatur-Berechnungseinheit (23), die an der Leiterplatte (20) montiert und so ausgeführt ist, dass sie Temperatur-Informationen der Leiterplatte (20) auf Basis des Temperatur-Reaktionssignals berechnet; sowie
eine Einheit (23) zum Korrigieren von Konzentrations-Informationen, die an der Leiterplatte (20) montiert und so ausgeführt ist, dass sie die **durch** die Einheit (26, 27, 22) zum Ansteuern des Erfassungselementes berechneten Gaskonzentrations-Informationen auf Basis der **durch** die Temperatur-Berechnungseinheit (23) berechneten Temperatur-Informationen korrigiert,

wobei sich das Temperatur-Messelement (29) näher an der Einheit (26, 27, 22) zum Ansteuern des Erfassungselementes als an der Einheit (28) zum Ansteuern des Heizelementes befindet oder das Temperatur-Messelement (29) innerhalb der Einheit (26, 27, 22) zum Ansteuern des Erfassungselementes angeordnet ist.

**2.** Sensor-Steuerungsvorrichtung nach Anspruch 1,
wobei der Gassensor (10) ein NOx-Sensor ist, der so ausgeführt ist, dass er NOx-Konzentration als eine erste bestimmte Gaskomponente misst, die in einem zu messenden Gas enthalten ist, und wobei das Erfassungselement umfasst:

eine erste Erfassungskammer (150), in die das zu messende Gas über einen ersten Diffusions-Verhinderungsabschnitt (151) eingeleitet wird;
eine erste Sauerstoff-Pumpzelle (110), die einen ersten Festelektrolyt-Körper (111) sowie ein Paar erster Elektroden (112, 113) umfasst, die an dem ersten Festelektrolyt-Körper ausgebildet sind, wobei eine des Paars erster Elektroden im Inneren der ersten Erfassungskammer angeordnet ist und die erste Sauerstoff-Pumpzelle so ausgeführt ist, dass sie Sauerstoff in das in die erste Erfassungskammer (150) eingeleitete zu messende Gas pumpt;
eine zweite Erfassungskammer (160), in die Gas aus der ersten Kammer über einen zweiten Diffusions-Verhinderungsabschnitt (152) eingeleitet wird; und
eine zweite Sauerstoff-Pumpzelle (130), die einen zweiten Festelektrolyt-Körper (131) sowie ein Paar zweiter Elektroden (132) umfasst, die an dem zweiten Festelektrolyt-Körper ausgebildet sind, wobei eine des Paars zweiter Elektroden im Inneren der zweiten Erfassungskammer (160) angeordnet ist,

und das Konzentrations-Reaktionssignal ein erstes Konzentrations-Reaktionssignal ist, das auf Basis von Strom erzeugt wird, der durch die zweite Sauerstoff-Pumpzelle (130) in Reaktion auf die Konzentration von NOx in der zweiten Erfassungskammer (160) fließt.

**3.** Sensor-Steuerungsvorrichtung nach Anspruch 2,
wobei sich die Einheit zum Ansteuern des Erfassungselementes separat von der Einheit zum Ansteuern des Heizelementes auf der Leiterplatte befindet;
und die Einheit zum Ansteuern des Erfassungselementes umfasst:

eine erste Zellen-Steuerungseinheit (26), die so ausgeführt ist, dass sie eine Spannung an die erste Sauerstoff-Pumpzelle anlegt, um der ersten Sauerstoff-Pumpzelle (110) Strom zuzuführen; und
eine zweite Zellen-Steuerungseinheit (27), die weiter von der Einheit (28) zum Ansteuern des Heizelementes entfernt ist als die erste Zellen-Steuerungseinheit, und so ausgeführt ist, dass sie eine Spannung an die zweite Sauerstoff-Pumpzelle anlegt, um der zweiten Sauerstoff-Pumpzelle (130) Strom zuzuführen.

**4.** Sensor-Steuerungsvorrichtung nach Anspruch 2 oder 3,
wobei das Konzentrations-Reaktionssignal zusätzlich zu dem ersten Konzentrations-Reaktionssignal ein zweites Konzentrations-Reaktionssignal umfasst, und
wobei, wenn Sauerstoff durch die erste Sauerstoff-Pumpzelle (110) in das oder aus dem in die erste Erfassungskammer (150) eingeleitete/n zu messende Gas gepumpt wird, das zweite Konzentrations-Reaktionssignal auf Basis des durch die erste Pumpzelle fließenden Stroms in Reaktion auf die Konzentration von Sauerstoff als eine zweite bestimmte Gaskomponente erzeugt wird, die in dem zu messenden Gas enthalten ist.

**5.** Sensor-Steuerungsvorrichtung nach einem der Ansprüche 1 bis 4,
wobei das Temperatur-Messelement (29) mit der Leiterplatte (20) in Kontakt ist, um die Temperatur der Leiterplatte (20) zu erfassen.

**Revendications**

**1.** Dispositif de commande de capteur comprenant :

une carte à circuit imprimé (20) séparée d'un capteur de gaz (10) et pouvant être reliée à celui-ci, ledit capteur de gaz (10) comprenant un élément de détection (100) configuré pour délivrer en sortie un signal de réponse de concentration en réponse à la concentration d'une composante de gaz spécifique, ainsi qu'un élément chauffant (180) configuré pour activer l'élément de détection,
une unité d'attaque d'élément de détection (26, 27, 22) montée sur la carte à circuit imprimé (20), l'unité d'attaque d'élément de détection (26, 27, 22) étant configurée pour :

commander le capteur de gaz (10),

calculer des informations de concentration de gaz sur la base du signal de réponse de concentration, et délivrer en sortie les informations calculées de concentration de gaz vers un circuit externe, et une unité d'attaque d'élément chauffant (28) montée sur la carte à circuit imprimé (20) et configurée pour fournir un courant d'attaque à l'élément chauffant (180),

**caractérisé par :**

un élément de détection de température (29) monté sur la carte à circuit imprimé (20) et configuré pour délivrer en sortie un signal de réponse en température en réponse à la température de la carte à circuit imprimé (20), une unité de calcul de température (23) montée sur la carte à circuit imprimé (20) et configurée pour calculer des informations sur la température de la carte à circuit imprimé (20) sur la base du signal de réponse en température, et

une unité de correction d'informations de concentration (23) montée sur la carte à circuit imprimé (20) et configurée pour corriger les informations sur la concentration de gaz calculées par l'unité d'attaque d'élément de détection (26, 27, 22) sur la base des informations de température calculées par l'unité de calcul de température (23),

dans lequel l'élément de détection de température (29) est situé pour être plus proche de l'unité d'attaque d'élément de détection (26, 27, 22) que de l'unité d'attaque d'élément chauffant (28), ou bien l'élément de détection de température (29) est agencé à l'intérieur de l'unité d'attaque d'élément de détection (26, 27, 22).

2. Dispositif de commande de capteur selon la revendication 1,
dans lequel le capteur de gaz (10) est un capteur de NOx configuré pour détecter la concentration en NOx comme première composante de gaz spécifique contenue dans le gaz à mesurer, l'élément de détection comprenant :

une première chambre de détection (150) dans laquelle le gaz à mesurer est introduit par l'intermédiaire d'une première partie résistant à la diffusion (151),
une première cellule de pompage d'oxygène (110) comprenant un premier corps d'électrolyte solide (111) et une paire de premières électrodes (112, 113) formées sur le premier corps d'électrolyte solide, l'une de la paire de premières électrodes étant agencée à l'intérieur de la première chambre de détection, la première cellule de pompage d'oxygène étant configurée pour pomper l'oxygène dans le gaz à mesurer introduit dans la première chambre de détection (150), ou hors de celui-ci,
une seconde chambre de détection (160) dans laquelle le gaz issu de la première chambre est introduit par l'intermédiaire d'une seconde partie résistant à la diffusion (152), et
une seconde cellule de pompage d'oxygène (130) comprenant un second corps d'électrolyte solide (131) et une paire de secondes électrodes (132) formées sur le second corps d'électrolyte solide, l'une de la paire de secondes électrodes étant agencée à l'intérieur de la seconde chambre de détection (160),

dans lequel le signal de réponse de concentration est un premier signal de réponse de concentration obtenu sur la base du courant circulant au travers de la seconde cellule de pompage l'oxygène (130) en réponse à la concentration de NOx dans la seconde chambre de détection (160).

3. Dispositif de commande de capteur selon la revendication 2,
dans lequel l'unité d'attaque d'élément de détection est située sur la carte à circuit imprimé séparée de l'unité d'attaque d'élément chauffant,
dans lequel l'unité d'attaque d'élément de détection comprend :

un premier circuit de commande de cellule (26) configuré pour appliquer une tension à la première cellule de pompage d'oxygène de sorte à fournir un courant à la première cellule de pompage d'oxygène (110), et
un second circuit de commande de cellule (27) situé plus loin de l'unité d'attaque d'élément chauffant (28) que du premier circuit de commande de cellule et configuré pour appliquer une tension à la seconde cellule de pompage d'oxygène de sorte à fournir du courant à la seconde cellule de pompage d'oxygène (130).

4. Dispositif de commande de capteur selon la revendication 2 ou la revendication 3,
dans lequel le signal de réponse de concentration inclut un second signal de réponse de concentration en plus du premier signal de réponse de concentration, et
dans lequel, lorsque l'oxygène est pompé dans le gaz à mesurer introduit dans la première chambre de détection (150) par la première cellule de pompage d'oxygène (110), ou hors de celui-ci, le second signal de réponse de concentration est obtenu sur la base du courant circulant au travers de la première cellule de pompage en réponse à la concentration oxygène en tant que seconde composante spécifique de gaz contenue dans le gaz à mesurer.

5. Dispositif de commande de capteur selon l'une quelconque des revendications 1 à 4,
   dans lequel l'élément de détection de température (29) est en contact avec la carte à circuit imprimé (20) pour détecter la température de la carte à circuit imprimé (20).

## FIG. 1

FIG. 2

EP 2 056 099 B1

## FIG. 3A

## FIG. 3B

# FIG. 4

```
          ┌────────────────────────────────┐
          │  NOx CONCENTRATION CORRECTING  │
          │        PROCESS OPERATION       │
          └────────────────────────────────┘
                          │
                          ▼                    S11
          ┌────────────────────────────────┐
          │     ACQUIRE TEMPERATURE        │
          │       DETECTION VALUE          │
          └────────────────────────────────┘
                          │
                          ▼                    S12
          ┌────────────────────────────────┐
          │  CONVERT DETECTION VALUE INTO   │
          │     TEMPERATURE INFORMATION     │
          └────────────────────────────────┘
                          │
                          ▼        S15
                   ╱─────────────╲        YES                              S16
                  ╱  HIGHER THAN   ╲──────────────────────┐   ┌──────────────────────────┐
                  ╲     35°C?      ╱                       └──▶│  CORRECT NOx CONCENTRATION │
                   ╲─────────────╱                            │     AT HIGH TEMPERATURE     │
                          │ NO                                └──────────────────────────┘
                          │
                          ▼        S20
                   ╱─────────────╲        YES
                  ╱  LOWER THAN    ╲──────────┐
                  ╲     35°C?      ╱          │                    S21
                   ╲─────────────╱           ▼    ┌──────────────────────────┐
                          │ NO                     │  CORRECT NOx CONCENTRATION │
                          │                        │     AT LOW TEMPERATURE      │
                          │                        └──────────────────────────┘
                          ▼
                   ┌─────────────┐
                   │   RETURN    │
                   └─────────────┘
```

FIG. 5

# FIG. 6

```
┌──────────────────────────────┐
│  NOx CONCENTRATION CORRECTING │
│      PROCESS OPERATION        │
└──────────────────────────────┘
              │
              ▼                    S11
┌──────────────────────────────┐
│     ACQUIRE TEMPERATURE       │
│       DETECTION VALUE         │
└──────────────────────────────┘
              │
              ▼                    S12
┌──────────────────────────────┐
│  CONVERT DETECTION VALUE INTO │
│    TEMPERATURE INFORMATION    │
└──────────────────────────────┘
              │
              ▼                    S15
        ◇─────────────◇    YES
      ╱  HIGHER THAN    ╲ ──────────────────────┐
      ╲    35°C?        ╱                        │
        ◇─────────────◇                          ▼                S17
              │ NO                  ┌──────────────────────────────┐
              │                     │ DERIVE CORRECTION COEFFICIENT │
              │                     │   OF O2 CONCENTRATION AT HIGH  │
              │                     │         TEMPERATURE           │
              │                     └──────────────────────────────┘
              │                                  │
              ▼                    S20           ▼                S18
        ◇─────────────◇    YES     ┌──────────────────────────────┐
      ╱  LOWER THAN     ╲ ───┐      │   DERIVE COEFFICIENT OF NOx    │
      ╲    35°C?        ╱     │     │    CONCENTRATION AT HIGH       │
        ◇─────────────◇      │     │         TEMPERATURE           │
              │ NO            │     └──────────────────────────────┘
              │               │
              │               ▼           S22
              │     ┌──────────────────────────────┐
              │     │ DERIVE CORRECTION COEFFICIENT │
              │     │   OF O2 CONCENTRATION AT LOW   │
              │     │         TEMPERATURE           │
              │     └──────────────────────────────┘
              │               │
              │               ▼           S23
              │     ┌──────────────────────────────┐
              │     │   DERIVE COEFFICIENT OF NOx    │
              │     │    CONCENTRATION AT LOW        │
              │     │         TEMPERATURE           │
              │     └──────────────────────────────┘
              │               │
              ▼◄──────────────┘
              │                    S25
┌──────────────────────────────┐
│    CALCULATE O2 CONCENTRATION  │
└──────────────────────────────┘
              │
              ▼                    S26
┌──────────────────────────────┐
│    CALCULATE TEMPORARY NOx     │
│        CONCENTRATION          │
└──────────────────────────────┘
              │
              ▼                    S27
┌──────────────────────────────┐
│     CALCULATE FINAL NOx        │
│        CONCENTRATION          │
└──────────────────────────────┘
              │
              ▼
        ┌─────────┐
        │  RETURN │
        └─────────┘
```

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10288595 A

- JP 2007280993 A